# EUROPEAN PATENT APPLICATION

(11) **EP 4 317 426 A1**
(43) Date of publication of application: **07.02.2024**
(21) Application number: 22188887.8
(22) Date of filing: 04.08.2022
(51) Int. Cl.: C12N 9/04, C12N 9/06, C12N 9/10, C12N 9/88, C12P 13/04, C12R 1/19

(54) **NEW CHANNEL FOR GLYCINE SYNTHESIS**

(71) Applicant: Dynveo, 34820 Teyran (FR)
(72) Inventor: WERHMANN, Matthias, 31300 Toulouse (FR); FRANCOIS, Jean Marie, 31830 Plaisance du Touch (FR); DUBOIS, Cécile, 34790 Grabels (FR); GUEDON, Rémi, 34820 TEYRAN (FR)
(74) Representative: Germain Maureau

(57) **Abstract**

The present invention concerns a metabolically engineered microorganism for glycine bioproduction or a salt or an ester thereof, the genome of said microorganism comprises an attenuation of the expression of genes encoding enzymes having glycine cleavage system activity as defined by E.C. 1.4.1.27 together with an overexpressing of threonine dehydrogenase dependent pathway as defined by EC E.C. 1.1.1.103 and E.C. 2.3.1.29 and/or with a threonine aldolase dependent pathway as defined by E.C. 4.1.2.48 or EC 4.1.2.42 or any of its catalytically active variants, its use for the production of glycine or one of its salts or esters. The present invention also concerns a fermentation process using said metabolically engineered microorganism for the production of glycine or one of its salts or esters.

## Description

### FIELD OF THE INVENTION

The invention relates to the biotechnology industry and more specifically to a metabolically engineered microorganism for the production of glycine or one of its salts or esters, and the use of said metabolically engineered microorganisms. The invention also relates to a fermentation process using said metabolically engineered microorganism for the production of glycine or one of its salts or esters.

### STATE OF THE ART

Glycine is one of the twenty-two proteinogenic amino acids. It is the simplest of the alpha-amino acids, whose chemical formula is C₂H₅NO₂. This amino acid has many metabolic roles and is also the precursor for the synthesis of many compounds. For example, glycine makes up one third of the amino acids content in collagen molecule, it can also combine with bile acids to make bile salts. Moreover, glycine is one of the limiting factors in the biosynthesis of glutathione, known for its role as a major antioxidant, participates in the hemoglobin synthesis and creatine metabolism. Glycine can also function as neurotransmitter, specifically in glutaminergic neurotransmission, which is important for cognition and sleep quality.

Studies also show that this amino acid can mitigate the harmful effects of a derivative of methionine in cardiovascular diseases and could be involved in increased longevity. Epidemiological studies have demonstrated an inverse correlation of plasma glycine levels with acute myocardial infarction as well as prevalence of diabetes, hypertension and obesity (Ding *et al.,* 2015). Moreover, glycine is widely in the food industry for its preservative properties, as a flavor enhancer due to its sweet nature or in animal nutrition. Glycine is also used in the pharmaceutical industry or in phytosanitary industry, e.g. pesticide and herbicide industry. For example, China is the world's main glycine producer with an annual output of 600 KT in 2014, with more than 80% being used for glyphosate production (Zeng et al., 2016). According to a review by Wendisch, the annual production of glycine in the feed and food industries in 2018 was estimated at 22,000 tons per year (Wendisch, 2019).

Nevertheless, and whatever the industrial segment considered (i.e. chemical, food, animal feed, cosmetic, pharmaceutical), glycine is the last amino acid produced solely by chemical synthesis from precursors of fossil origin (Tonouchi *et al.,* 2016). There are different chemical processes that have been developed for the synthesis of this non chiral amino acid at the industrial scale. The process most frequently used in industry is the amination of monochloroacetic acid in the presence of ammonia and chemical catalysts giving glycine and ammonium chloride (Orten and Hill, 1931). Glycine synthesis by the Strecker method (Strecker, 1850) which allows the synthesis of this amino acid by the reaction of an aldehyde with ammonium chloride in the presence of potassium cyanide is the other well-known industrial process. A third process corresponds to a hydrolysis of hydantoin in the presence of organic solvents (Boyd and Robson, 1935). However, this hydrolysis process has the disadvantage that hydantoin is synthesized from hydrogen cyanide and formaldehyde, two particularly toxic products classified as carcinogens, mutagens and toxic to reproduction.

Given the major interest in glycine, particularly in the food and pharmaceutical market, other synthesis processes have been developed but are less used. By way of example, the synthesis of glycine has been described from a reductive amination of glyoxylate in the presence of rhodium as a catalyst, a chemical synthesis process from glycinonitrile, ammonia and carbon dioxide or even a chemical synthesis method using potassium phthalimide and monochloroacetic acid to produce glycine and phthalic acid.

The increased problem of pollution combined with a constant increase in the emission of greenhouse gases and an increase in the world population force us to revise the economic model based on fossil resources. Thus, one of the major challenges facing society is the transition from an economy based on the use of materials and energy of petro-sourced origin to their equivalents derived from renewable biomass.

It emerges from the foregoing that there is an obvious need to develop new technical solutions allowing a production of glycine responding to the aspiration of consumers to use products of biological origin, to acquire an eco-responsible behavior and to limit its environmental impact. Thus, there remains an obvious need to develop an effective production of glycine by biological means, in particular at an industrial scale, with a level of purity sufficient to meet the needs of the agro-food and pharmaceutical industries and at a reasonable price.

### DESCRIPTION OF THE INVENTION

The inventors have developed, unexpectedly and surprisingly, metabolically engineered microorganisms, that are able to ensure efficient production of glycine, or a salt or an ester thereof, by fermentation of a suitable culture medium comprising a carbon source, in particular a simple carbon source. These metabolically engineered microorganisms or recombinant microorganisms according to the invention, their uses and the method for producing glycine, or a salt or an ester thereof, by fermentation are described throughout the present description.

An objective of the present invention is to produce glycine using a biological system as a cell factory. This objective is achieved in particular *via* the overexpression of the glycine production pathways and the attenuation or suppression of the glycine degradation pathway.

Thus, another objective of the present invention is to produce glycine biologically from renewable carbon sources using microorganisms, which may have advantages over environmentally unfriendly chemical processes, enable to reduce toxic co-products and energy costs of chemical processes.

The present invention relates to a metabolically engineered microorganism for glycine bioproduction or a salt or an ester thereof, in particular from threonine as a direct precursor, the genome of which comprises:
(A) Attenuation of the expression of genes encoding enzymes having glycine cleavage system activity as defined by EC 1.4.1.27, in particular enzymes having glycine decarboxylase activity as defined by EC 1.4.4.2 and an aminomethyltransferase activity as defined by EC 2.1.2.10; and
(B) Overexpression of genes encoding enzymes having L-threonine 3-dehydrogenase activity as defined by EC 1.1.1.103 and a glycine c-acetyltransferase activity as defined by EC 2.3.1.29; and/or
(C) Overexpression of the gene encoding an enzyme having L-threonine aldolase activity as defined by EC 4.1.2.48, or a variant of this enzyme , EC 4.1.2.42 or EC 4.1.2.49.

According to the invention, the loss of activity of the glycine cleavage system, encoded by the *gcv* operon, is accompanied by an excretion of glycine into the medium (Plamann, 1983). Thus, an efficient production of glycine from threonine as a substrate is obtained by combining this modification with:
- the overexpression of threonine degradation pathway I (termed TDGI), corresponding to the gene coding for the enzyme having an L-threonine 3-dehydrogenase, which converts threonine to L-2-amino-oxobutanoate and the gene encoding the enzyme with glycine c-acetyltransferase activity (or 2-amino-3-ketobutyrate coenzyme A ligase activity) which converts L- 2-amino-oxobutanoate to glycine and acetyl-CoA; and/or
- the overexpression of the threonine degradation pathway II (TDGII) which corresponds to the gene encoding a L-threonine aldolase which cleaves threonine into glycine and acetaldehyde.

To enhance the glycine yield, the overexpression of a gene encoding either an enzyme having L- or D-amino acid dehydrogenase activity as defined by EC 1.4.99.1. or EC 1.4.1.9, respectively, or an enzyme with glyoxylate-alanine transaminase activity allows the transformation of glyoxylate into glycine, which is obtained from acetyl-COA formed as by-products in threonine degradation pathways I and II.

In the context of the invention, the expressions "metabolically engineered microorganism", "recombinant microorganism" and "genetically modified microorganism" are used interchangeably and mean that the microorganism according to the invention is not found in nature and is modified by introduction of new genetic elements and/or by deletion or modification of endogenous genetic elements of the microorganism. Such a microorganism can be subjected to selection pressure, by combining site-directed mutagenesis or genomic recombination and culturing in the selection medium.

In the context of the invention, enzymatic activities are also designated by reference to the genes encoding the enzymes having such an activity.

The use of gene designation is not limited to a specific organism, but covers all corresponding genes and proteins in other organisms (e.g. microorganisms, functional analogs, functional variants and their functional fragments (as long as they retain enzymatic activity).

In the context of the invention, the term "precursor" designates an initial or intermediate production substrate that is enzymatically converted into a product in order to, *in fine,* produce glycine.

In the context of the invention, the term "endogenous gene" means that the gene was present in the microorganism before any genetic modification, in the wild-type strain. Endogenous genes can be overexpressed by introducing heterologous sequences in addition to or replacing endogenous regulatory elements, or by replacing its own promoter by a strongest one, or by introducing one or more additional copies of the gene into the chromosome or a plasmid. Endogenous genes can also be modified in order to modulate their expression. For example, mutations can be introduced into the promoter sequence to modify expression, or heterologous sequences can be introduced in addition to or to replace endogenous regulatory elements. Modulation of an endogenous gene can result in upregulation and/or enhancement of gene product activity, or alternatively, downregulation and/or attenuation of endogenous gene product activity. Another way to enhance the expression of endogenous genes is to introduce one or more additional copies of the gene onto the chromosome or a plasmid. Conversely, attenuation of expression of endogenous genes can be obtained by deletion of that gene from the chromosome.

In the context of the invention, the term "heterologous gene" or "exogenous gene" are used interchangeably and means that the gene has been introduced into a microorganism, by means well known to those skilled in the art, whereas this gene is not naturally present in the wild-type microorganism. A microorganism can express exogenous genes if these genes are introduced into the microorganism with all the elements allowing their expression in the host microorganism. Transforming microorganisms with exogenous DNA is a routine task for those skilled in the art. Exogenous genes can be integrated into the host chromosome or be expressed extrachromosomally *via* plasmids or vectors. A variety of plasmids, which differ in their origin of replication and their copy number in the cell, are all known in the art (e.g. see Dykxhoorn *et al.,* 1996 or Woodall, 2003). The sequence of exogenous genes can be adapted for its expression in the host microorganism. Indeed, those skilled in the art know the concept of codon usage bias and how to adapt nucleic acid sequences for a particular codon usage bias without modifying the deduced protein.

In the context of the invention, the term "overexpression" means that the expression of a gene or of a protein, e.g. an enzyme, is increased compared to the unmodified microorganism. The increase in the expression of an enzyme is obtained by increasing the expression of a gene encoding said enzyme. The increase in the expression of a gene can be carried out by all the techniques known to those skilled in the art. In this respect, mention may in particular be made of the implementation of a strong promoter upstream of the nucleic acid intended to be overexpressed or the introduction into the genome of several copies of said nucleic acid between a promoter, in particular a strong promoter, and a terminator.

In the context of the invention, the terms "attenuation", "under expression" and "repression" are used interchangeably and mean that the expression of a gene or of a protein, e.g. an enzyme, is reduced, or even completely inhibited/suppressed, compared to the wild type, i.e. no protein is produced, in particular a functionally effective protein (e.g. enzyme with enzymatic activity), or the protein is produced but is not functional/not active. The decrease, attenuation or repression in the expression of an enzyme is obtained by decreasing or inhibiting the expression of a gene coding for said enzyme. The reduction, attenuation or repression in the expression of a gene can be carried out by all the techniques known to those skilled in the art. In this respect, mention may in particular be made of the implementation of a weak promoter upstream of the coding sequence of the gene intended to be under-expressed. Mention may also be made of the use of a nucleic acid encoding a variant of said enzyme that is less active than the original enzyme or a variant of said enzyme that is more rapidly degraded in the cell than the original enzyme. Variants of an original enzyme which degrade faster than said parent enzyme include degron-tagged enzymes. These degron-tagged enzymes include an added protein degradation signal amino acid sequence that serves as a kill signal that will cause said enzyme to undergo degradation, which can be either (i) degradation independent of ubiquitin or (ii) ubiquitin-dependent degradation. Mention may also be made of the reduction in the expression of a transcription activator of the gene of interest.

In the context of the invention, the term "inducible promoter" is used to qualify a promoter whose activity is induced, that is to say increased:
- in the presence of one or more particular metabolite(s). The higher the metabolite concentration in the medium, the stronger the promoter activity; or
- in the presence of a low concentration, or in the absence, of one or more metabolite(s). These metabolites are different from those whose increasing presence induces the activity of the promoter. The lower the metabolite concentration in the medium, the stronger the promoter activity.

In the context of the invention, the term "repressible promoter" is used to qualify a promoter whose activity is repressed, that is to say reduced:
- in the presence of one or more particular metabolite(s). The higher the metabolite concentration in the medium, the weaker the promoter activity; or
- in the presence of a low concentration, or in the absence, of one or more metabolite(s). These metabolites are different from those whose increasing presence represses promoter activity. The lower the metabolite concentration in the medium, the weaker the promoter activity.

In the context of the invention, the term "activity" of an enzyme is used interchangeably with the term "function" and designates the ability of an enzyme to catalyze a desired reaction.

The terms "reduced activity" or "attenuated activity" of an enzyme means either a reduced, or even a suppressed, specific catalytic activity of the enzyme obtained by mutation in the amino acid sequence and/or a decrease in the concentrations of the enzyme in the cell obtained by mutation of the nucleotide sequence or by deletion of the corresponding related gene or else by degron-labeling of the enzyme.

The term "increased activity" of an enzyme means either an increased specific catalytic activity of the enzyme, and/or an increased quantity/availability of the enzyme in the cell, obtained for example by overexpression of the gene encoding the enzyme .

In the context of the invention, the term "variant" or "functional variant" encompasses enzymes which may exhibit substantial sequence modifications compared to the sequences specifically described in the present application but which still retain the original enzymatic activity. This also means that the sequence of the enzyme may comprise fewer amino acids than the original one but that said truncated enzyme still retains the original enzymatic activity.

In the context of the invention, the articles "a" and "an" are used to refer to one or more (for example at least one) units of the grammatical object of the article. By way of example, "an element" designates at least one element, that is to say one or more elements.

The terms "about" or "approximately", used in reference to a measurable value such as a quantity, a duration, and other analogous values, must be understood as encompassing measurement uncertainties of ± 20% or ± 10%, preferably ± 5%, even more preferably ± 1%, and particularly preferably ± 0.1% of the specified value.

The term "isolated" should be understood in the context of the invention as synonymous with removed or extracted from its environment or natural state. For example, an isolated nucleic acid or peptide is a nucleic acid or peptide extracted from the natural environment in which it is usually found, be it a living plant or animal for example. Thus, a nucleic acid or a peptide naturally present in a living animal is not an isolated nucleic acid or peptide within the meaning of the invention, while the same nucleic acid or peptide, partially or completely separated from the other elements present in its natural context, is itself "isolated" within the meaning of the invention. An isolated nucleic acid or peptide may exist in a substantially purified form, or may exist in a non-native environment such as, for example, a host cell.

In the context of the invention, a "nucleotide sequence coding for an amino acid sequence" designates all the nucleotide sequences which code for the amino acid sequence, including the degenerate nucleotide sequences making it possible to obtain said sequence of amino acid. The nucleotide sequence which encodes a protein or an RNA or a cDNA may optionally include introns.

In the context of the invention, the terms "coding" or "coding for", "code" or "code for" refer to the property inherent in the specific sequences of nucleotides in a polynucleotide, such as a gene, a cDNA or an mRNA, to serve as a template for the synthesis of other polymers and macromolecules in biological processes, having either a defined sequence of nucleotides (e.g. rRNA, tRNA and mRNA), or a defined sequence of amino acids , and the resulting biological properties. Thus, a gene encodes a protein if transcription and translation of the mRNA corresponding to that gene produce the protein in a cell or other biological system. Both the coding strand, whose nucleotide sequence is identical to the mRNA sequence and which is generally described in sequence listings and databases, and the noncoding strand, used as a template for the transcription of a gene or cDNA, may be referred to as coding for the protein or other product of that gene or cDNA.

In the context of the invention, the term "polynucleotide" is defined as a chain of nucleotides. Also, nucleic acids are polymers of nucleotides. Thus, the terms nucleic acids and polynucleotides as used in the context of the invention are interchangeable. It is well known in the field of molecular biology and genetic engineering that nucleic acids are polynucleotides, which can be hydrolyzed into monomers. Nucleotides in monomeric form can be hydrolyzed into nucleosides. As used in the context of the invention, the term polynucleotide denotes, without limitation, any type of nucleic acid molecule, that is to say nucleic acid molecules obtainable by any means available in the art, including by recombinant means, namely the cloning of nucleic acid sequences from a recombinant library or the genome of a cell, using ordinary cloning technologies such as PCR, or by synthesis.

Within the meaning of the invention, the terms "peptide", "polypeptide", "protein" and "enzyme" are used interchangeably and refer to a compound consisting of amino acid residues covalently linked by peptide bonds. A protein by definition contains at least two amino acids, with no limitation as to the maximum number of amino acids. An enzyme is a protein that catalyzes a biochemical reaction, especially in a cell. The polypeptides include without distinction several peptides and/or proteins, which themselves include two or more amino acids connected to each other by peptide bonds. As used herein, the term refers both to short chains, which are also commonly referred to in the art as peptides, oligopeptides and oligomers for example, and longer chains, which are generally referred to in the art as proteins, of which there are many types. "Polypeptides" include, for example, biologically active fragments, substantially homologous polypeptides, oligopeptides, homodimers, heterodimers, variant polypeptides, modified polypeptides, derivatives, analogs, fusion proteins, among others. Polypeptides include natural peptides, recombinant peptides, synthetic peptides, or a combination thereof.

In the context of the invention, the terms "homologous" and "identical" refer to the sequence similarity or the sequence identity between two polypeptides or between two nucleic acid molecules. When a position in each of the two compared sequences is occupied by the same base or monomeric amino acid subunit (for example, when a position in each of the two DNA molecules is occupied by an adenine), then the molecules are homologous or identical for this position. The percentage identity between two sequences is a function of the number of corresponding positions shared by the two sequences, and corresponds to this number divided by the number of positions compared and multiplied by 100. For example, if 6 out of 10 of the positions in two sequences matched are identical, then the two sequences are 60% identical. Typically, the comparison is made by aligning the two sequences to give maximum identity/homology.

In the context of the invention, a "vector" is a molecular construct which comprises an isolated nucleic acid and which can be used to deliver the isolated nucleic acid inside a cell. Many vectors are known in the art including, but not limited to, linear polynucleotides, polynucleotides associated with ionic or amphiphilic compounds, plasmids and viruses. Thus, the term "vector" denotes, for example, an autonomously replicating plasmid or a virus. The term is also to be construed to include non-plasmid or non-viral compounds which facilitate the transfer of nucleic acids into cells, such as, for example, polylysine compounds, liposomes, and the like.

In the context of the invention, the terms "expression vector" denote a vector comprising a recombinant polynucleotide, which comprises expression control sequences operably linked to a nucleotide sequence to be expressed. An expression vector notably comprises cis-acting expression elements; other elements for expression which may be provided by the host cell or by an in vitro expression system. Expression vectors within the meaning of the invention include all those known in the art, such as cosmids, plasmids (for example naked or contained in liposomes) and viruses (for example lentiviruses, retroviruses, adenoviruses and adeno-associated viruses) which incorporate the recombinant polynucleotide.

In the context of the invention, the term "promoter" is defined as a DNA sequence recognized by the synthetic machinery of the cell, or the synthetic machinery introduced, necessary to initiate the specific transcription of a sequence of polynucleotides.

Within the meaning of the invention, the terms "promoter/regulatory sequence" designate a nucleic acid sequence, necessary for the expression of the polynucleotide operationally linked to the promoter/regulatory sequence. In some cases, this sequence may be the base sequence of the promoter, while in other cases this sequence may also include an enhancer sequence and other regulatory elements, useful for the expression of the polynucleotide. The promoter/regulatory sequence can be, for example, a sequence allowing the expression of the polynucleotide which is specific for a tissue, that is to say occurring preferentially in this tissue.

In the context of the invention, a "constitutive" promoter is a nucleotide sequence which, when operably linked to a polynucleotide, leads to expression of the polynucleotide under most or all physiological conditions of the cell.

In the context of the invention, the term "heterologous expression" designates the expression of exogenous genes in a host cell or organism if these are introduced into said cell or said organism with all the elements allowing their expression in this host. The techniques allowing the introduction of DNA into a host (or transformation) are well known to those skilled in the art and include in particular the permeabilization of membranes by application of an electric field (electroporation), by thermal means (application of thermal shock) or by chemical means.

Preferably, the subject of the present invention is a recombinant microorganism for the production of glycine or one of its salts or esters as defined above having the following technical features, taken alone or in combination:
- threonine is L-threonine, D-threonine or a mixture thereof;
- the genome of the microorganism of the invention further comprises the attenuationof the expression of the gene encoding an enzyme having a dihydrolipoyl dehydrogenase activity as defined by EC 1.8.1.4, advantageously it is an attenuation of its expression but not a complete inhibition/repression of this expression so that a dihydrolipoyl dehydrogenase activity can be quantified/observed;
- the gene encoding an enzyme having a glycine decarboxylase activity as defined by EC 1.4.4.2 is suppressed;
- the gene encoding an enzyme having an aminomethyltransferase activity as defined by EC 2.1.2.10 is suppressed ;
- the genome of the microorganism of the invention further comprises the overexpression of the gene encoding the enzyme having acetylating aldehyde dehydrogenase activity as defined by EC 1.2.1.10, allowing the conversion of acetaldehyde to acetyl-CoA;
- the genome of the microorganism of the invention further comprises the attenuation, or even the suppression, of the gene encoding the enzyme having a D-amino acid oxidase activity as defined by E.C. 1. 4. 99.-.
- the genome of the microorganism of the invention further comprises the overexpression of the gene coding for the enzyme having D-serine/D-alanine/glycine transporter activity as defined by TCDB 2.A.3.1.7;
- the genome of the microorganism of the invention further comprises the attenuation, or even the suppression, of the expression of the genes coding for enzymes having an extracellular threonine efflux activity (i.e. RhtC) as defined by TCBD 2.A.76.1.2 in particular threonine/homoserine export activity (RhtA) as defined by TCDB 2.A.7.3.6, homoserine/homoserine lactone/β-hydroxynorvaline efflux permease activity (RhtB) as defined by TCDB 2.A.76.1.1;
- the genome of the microorganism of the invention further comprises the overexpression of the gene encoding the enzyme having threonine synthase activity as defined by EC 4.2.3.1;
- the genome of the microorganism of the invention further comprises the overexpression of the gene encoding the enzyme having a glycine dehydrogenase activity as defined by EC 1.4.1.10, as for instance the L-alanine dehydrogenase of *M*. *tuberculosis* (Usha et al., 2002);
- the genome of the microorganism of the invention further comprises the overexpression of the gene encoding the enzyme having an D-amino acid dehydrogenase activity as defined by EC 1.4.99.1.;
- the genome of the microorganism of the invention further comprises the overexpression of the enzyme having glyoxylate-alanine transaminase activity as defined by EC 2.6.1.44;
- the variant of the enzyme having an L-threonine aldolase activity as defined by EC:4.1.2.48 is the H126F variant of the constitutive enzyme of *Escherichia coli,* namely ItaEH126F as described in Fesko (Fesko, 2016); or an equivalent variant of another microorganism;
- the enzyme having glycine decarboxylase activity as defined by EC 1.4.4.2 is encoded by sequence set in forth in SEQ ID NO: 1 of *E. coli,* or any sequence sharing an identity of at least 90% with said sequence or corresponds to SEQ ID NO: 2 or any sequence sharing an identity of at least 90% with said sequence or corresponds to an equivalent enzyme having the same enzymatic activity in another microorganism;
- the enzyme having aminomethyltransferase activity as defined by EC 2.1.2.10 is encoded by sequence set in forth in SEQ ID NO: 3 of *E. coli,* or any sequence sharing an identity of at least 90% with said sequence or corresponds to SEQ ID NO: 4 or any sequence sharing an identity of at least 90% with said sequence or corresponds to an equivalent enzyme having the same enzymatic activity in another microorganism;
- the enzyme having a L-threonine 3-dehydrogenase activity as defined by EC 1.1.1.103 is encoded by sequence set in forth in SEQ ID NO: 5 of *E. coli,* or any sequence sharing an identity of at least 90% with said sequence or corresponds to SEQ ID NO: 6 or any sequence sharing an identity of at least 90% with said sequence or corresponds to an equivalent enzyme having the same enzymatic activity in another microorganism;
- the enzyme having a L-threonine aldolase activity as defined by EC 4.1.2.48 is encoded by sequence set in forth in SEQ ID NO: 7 of *E. coli,* or any sequence sharing an identity of at least 90% with said sequence or corresponds to SEQ ID NO: 8 or any sequence sharing an identity of at least 90% with said sequence or corresponds to an equivalent enzyme having the same enzymatic activity in another microorganism;
- the enzyme having dihydrolipoyl dehydrogenase activity as defined by EC 1.8.1.4 is encoded by sequence set in forth in SEQ ID NO: 9 of *E. coli,* or any sequence sharing an identity of at least 90% with said sequence or corresponds to SEQ ID NO: 10 or any sequence sharing an identity of at least 90% with said sequence or corresponds to an equivalent enzyme having the same enzymatic activity in another microorganism;
- the enzyme having an acetylating aldehyde dehydrogenase activity as defined by EC 1.2.1.10 is encoded by sequence set in forth in SEQ ID NO: 11 of *E. coli,* or any sequence sharing an identity of at least 90% with said sequence or corresponds to SEQ ID NO: 12 or any sequence sharing an identity of at least 90% with said sequence or corresponds to an equivalent enzyme having the same enzymatic activity in another microorganism;
- the enzyme having threonine synthase activity as defined by EC 4.2.3.1 is encoded by sequence set in forth in SEQ ID NO: 13 of *E. coli,* or any sequence sharing an identity of at least 90% with said sequence or corresponds to SEQ ID NO: 14 or any sequence sharing an identity of at least 90% with said sequence or corresponds to an equivalent enzyme having the same enzymatic activity in another microorganism;
- the enzyme having glycine dehydrogenase activity as defined by EC 1.4.1.10 is encoded by sequence set in forth in SEQ ID NO: 15 of *Streptomyces phoechromogenes,* or any sequence sharing an identity of at least 90% with said sequence or corresponds to SEQ ID NO: 16 or any sequence sharing an identity of at least 90% with said sequence or corresponds to an equivalent enzyme having the same enzymatic activity in another microorganism;
- the enzyme having D-amino acid dehydrogenase activity as defined by EC 1.4.99.1. is encoded by sequence set in forth in SEQ ID NO: 17 of *E. coli,* or any sequence sharing an identity of at least 90% with said sequence or corresponds to SEQ ID NO: 18 or any sequence sharing an identity of at least 90% with said sequence or corresponds to an equivalent enzyme having the same enzymatic activity in another microorganism;
- the enzyme having glyoxylate-alanine transaminase activity as defined by EC 2.6.1.44 is encoded by sequence set in forth in SEQ ID NO: 19 of *E. coli,* or any sequence sharing an identity of at least 90% with said sequence or corresponds to SEQ ID NO: 20 or any sequence sharing an identity of at least 90% with said sequence or corresponds to an equivalent enzyme having the same enzymatic activity in another microorganism;
- the H126F variant of the enzyme having L-threonine aldolase activity as defined by EC 4.1.2.48 corresponds to the amino acid sequence as shown in SEQ ID NO: 21 of *E. coli* or any sequence sharing an identity of at least 90% with said sequence or corresponds to SEQ ID NO: 22 or any sequence sharing an identity of at least 90% with said sequence or corresponds to an equivalent enzyme having the same enzymatic activity in another microorganism;
- the enzyme having glycine C-acetyltransferase activity as defined by EC 2.3.1.29 is encoded by sequence set in forth in SEQ ID NO: 23 of *E. coli,* or any sequence sharing an identity of at least 90% with said sequence or corresponds to SEQ ID NO: 24 or any sequence sharing an identity of at least 90% with said sequence or corresponds to an equivalent enzyme having the same enzymatic activity in another microorganism;
- the enzyme having D-serine/D-alanine/glycine transporter activity as defined by TCDB 2.A.3.1.7 is encoded by sequence set in forth in SEQ ID NO: 25 of *E. coli,* or any sequence sharing an identity of at least 90% with said sequence or corresponds to SEQ ID NO: 26 or any sequence sharing an identity of at least 90% with said sequence or corresponds to an equivalent enzyme having the same enzymatic activity in another microorganism;
- the enzyme having threonine/homoserine exporter activity as defined by TCDB 2.A.7.3.6 is encoded by sequence set in forth in SEQ ID NO: 27 of *E. coli,* or any sequence sharing an identity of at least 90% with said sequence or corresponds to SEQ ID NO: 28 or any sequence sharing an identity of at least 90% with said sequence or corresponds to an equivalent enzyme having the same enzymatic activity in another microorganism;
- the enzyme having homoserine/homoserine lactone/β-hydroxynorvaline efflux permease activity is encoded by sequence set in forth in SEQ ID NO: 29 of *E. coli,* or any sequence sharing an identity of at least 90% with said sequence or corresponds to SEQ ID NO: 30 or any sequence sharing an identity of at least 90% with said sequence or corresponds to an equivalent enzyme having the same enzymatic activity in another microorganism;
- the enzyme having threonine efflux permease activity as defined by TCDB 2.A.76.1.2 is encoded by sequence set in forth in SEQ ID NO: 31 of *E. coli,* or any sequence sharing an identity of at least 90% with said sequence or corresponds to SEQ ID NO: 32 or any sequence sharing an identity of at least 90% with said sequence or corresponds to an equivalent enzyme having the same enzymatic activity in another microorganism;
- the enzyme having L-amino acid dehydrogenase activity as defined by EC 1.4.1.9 is encoded by sequence set in forth in SEQ ID NO: 33 of *Bacillus subtilis,* or any sequence sharing an identity of at least 90% with said sequence or corresponds to SEQ ID NO: 34 or any sequence sharing an identity of at least 90% with said sequence or corresponds to an equivalent enzyme having the same enzymatic activity in another microorganism;
- the salt form of glycine is ammonium, potassium or sodium glycine salt;
- the ester derived from glycine is selected from the group consisting of glycine ethyl ester, glycine methyl ester, glycine ethyl ester hydrochloride and glycine methyl ester hydrochloride;- the microorganism is any lower unicellular organism into which the gene(s), nucleic acid(s) or chimeric vector(s) according to the invention can be introduced to produce glycine or one of its salts or esters;
- the microorganism is a bacteria, advantageously of the family *Enterobacteriaceae* or *Corynebacteriaceae,* preferably of the genus *Escherichia, Pantoea, Corynebacterium* or *Brevibacterium,* more particularly of the species *Escherichia coli, Pantoea ananatis* or *Corynebacterium glutamicum*;
- the microorganism is a fungi, advantageously from the *Ascomycota* family, preferably from the *Saccharomyceta* or *Taphrinomycotina* group, more particularly from the *Saccharomycotina, Pezizomycotina* or *Archaeorhizomycetes* subphylum, more advantageously from the *Saccharomycetes, Eurotiomycetes, Leotiomyceta, Pezizomycetes* or *Archaeorhizomycetales* class , preferentially of the species *Saccharomyces cerevisiae, Talaromyces versatilis, Aspergillus niger.*

In the context of the invention, the expression "any sequence sharing an identity of at least 90% with a sequence (nucleic or peptide sequence)" corresponds to any sequence sharing an identity of at least 90%, or 91%, 92%, 93%, 94%, 95% or even 96%, 97%, 98%, or 99% with said sequence.

In the context of the invention, the expression "equivalent enzyme in another microorganism" or "enzyme having the same enzymatic activity in another microorganism", means an enzyme of a first microorganism capable of catalyzing a reaction on a substrate, or a set of related reactions on a set of substrates, said enzyme of the first microorganism has the same enzymatic properties (reaction rate, specificity of the substrate, etc.), or related/similar properties, as an enzyme of a second microorganism different from said first microorganism. In other words, it involves the specific recognition of a substrate, in particular the active site or catalytic site, then its conversion into product(s), by an enzyme derived from a microorganism different from that from which said specific substrate is derived. In particular, in the context of the invention, this expression does not designate a double enzymatic specificity, nor coenzymes.

In particular, the synthetic pathway for the production of glycine according to the invention, as described previously, can be implemented in a microorganism with an increased production of threonine. For example, mention may be made of bacteria of the genus *Corynebacterium* or *Brevibacterium* sp, *genus Escherichia,* in particular *Escherichia coli.*

Alternatively, the engineered pathway for the production of glycine according to the invention, as described previously, can be implemented in a microorganism exhibiting an increased production of threonine or with low threonine production. For example, we can mention the bacteria *Escherichia coli* (Debabov, 2003), the yeast *Saccharomyces cerevisiae* (Farfan et al., 1999) , the filamentous fungi *Aspergillus niger, Trichoderma reesei* or *Talaromyces versatilis.* In this case, the genome of said microorganism of the invention is modified as described above but also to ensure production of threonine, in particular increased production of threonine, in the microorganisms *via*:
(i) overexpression of the enzymes aspartate kinase, aspartate semialdehyde dehydrogenase, homoserine dehydrogenase, homoserine kinase and threonine synthase;
(ii) modification of the enzyme aspartate kinase to render it insensitive to product inhibition which may be caused by lysine, methionine and/or threonine (i.e. modify said enzyme to make it insensitive to the negative feedback induced by lysine methionine and/or threonine), and modification of the enzyme homoserine kinase to render it insensitive to threonine inhibition (i.e. modify said enzyme to make it insensitive to the negative feedback induced by threonine), and
(iii) suppression of metabolic pathways that divert away the aspartate-homoserine biosynthetic pathway from threonine synthesis, such as attenuation of methionine pathway.

In particular, overexpression of aspartate kinase, aspartate semialdehyde dehydrogenase, homoserine dehydrogenase, homoserine kinase and threonine synthase can be achieved by expressing the enzymes from a multicopy plasmid under the control of a constitutive promoter or an appropriate inducible promoter. Alternatively, the overexpression of said enzymes can be obtained by expressing the concerned genes under strong and non-repressible promoters. Aspartate kinases can be rendered immune to inhibition by amino acids derived from aspartate by introducing appropriate mutations in their amino acid sequences (Omori *et al.,* (1993); Huo *et al.,* (1996); Chen *et al.,* (2011)). Homoserine kinase can be rendered insensitive to threonine by introducing appropriate mutations in their amino acid sequence. Entry points into metabolic pathways that hijack the homoserine biosynthetic pathway are catalyzed by enzymes with O-succinyl homoserine or O-acetyl homoserine synthase activity (entry into methionine biosynthesis) or diaminopimelate activity decarboxylase (entry into lysine biosynthesis). The deletion of the genes coding for the proteins having said enzymatic activities prevents the formation of amino acids derived from aspartate, lysine and/or methionine, and therefore facilitates the formation of homoserine. Consequently, deletion of the *metA, thrB* and *lysA* genes in *E. coli* (or the equivalent enzymes in other microorganisms) enables higher flux of homoserine towards threonine synthesis (i.e. attenuates or even inhibits the diversion of homoserine into competitive pathways). For example, the increase in the enzymatic activities of the threonine pathway in *E. coli* can be obtained by the overexpression of the gene encoding the bifunctional aspartate kinase-homoserine dehydrogenase mutant *ThrA^{S345F}* (insensitive to threonine inhibition), as well as *asd, thrB* and *thrC* or by overexpression of the gene encoding the monofunctional aspartate kinase mutant *LysC ^{E250K}* (lysine insensitive), *asd, thrB, thrC* (all *E. coli* genes) (Lee *et al.,* 2007;Dong *et al.,* 2012).

The microorganism of the invention may also have an attenuated capacity or an absence of capacity to export threonine, which increases the intracellular availability of this amino acid. For example, when the microorganism is *E. coli,* its genome may further comprise the deletion of the threonine efflux transporters *rhtA, rhtB* and/or *rhtC* (Kruse *et al.,* 2002).

According to the invention, acetyl-CoA which is a by-product of the reaction catalyzed by the enzyme having glycine C-acetyltransferase activity (or 2-amino-3-ketobutyrate coenzyme A ligase), is "recycled" / used for the production of glycine *via* glyoxylate, then requiring the activation of the glyoxylate shunt and the expression of an enzyme having glycine dehydrogenase activity as defined by EC 1.4.1.10 or an enzyme having glyoxylate-alanine transaminase activity as defined by EC 2.6.1.44, to ensure the conversion of glyoxylate to glycine through a D-amino acid dehydrogenase activity as defined by EC 1.4 99.- or a alanine-glyoxylate transaminase as defined by EC 2.6.1.44 or glutamate-glyoxylate aminotransferase defined by EC 2.6.1.4, or serine-glyoxylate aminotransferase as defined by EC 2.6.1.45

Acetyl-CoA can also be produced from acetaldehyde which is the co-product of the cleavage of threonine to glycine catalyzed by an enzyme having L-threonine aldolase activity as defined by EC 4.1.2.48 or one of the variants of this enzyme. In this case, the acetyl-CoA is "recycled"/used *via* a glyoxylate shunt and the expression of an enzyme with glycine dehydrogenase activity as defined by EC 1.4.1.10, or an enzyme having glyoxylate-alanine transaminase activity as defined by EC 2.6.1.44 or glutamate--glyoxylate aminotransferase defined by EC 2.6.1.4, or serine-glyoxylate aminotransferase defined by EC 2.6.1.45 to ensure the production of glycine.

The invention also relates to the use of a microorganism as described previously for the production and/or the secretion and/or the purification of glycine or a salt or ester thereof. In particular:
- the salt form of glycine is ammonium, potassium or sodium glycine salt; and
- the ester derived from glycine is selected from the group consisting of glycine ethyl ester, glycine methyl ester, glycine ethyl ester hydrochloride and glycine methyl ester hydrochloride.

Preferably, the present invention relates to the use of a metabolically engineered microorganism as described above in which glycine or one of its salts or esters is produced from a carbon source, advantageously a simple carbon source, preferably a pentose or a hexose or a disaccharide, more particularly chosen from the group consisting of glucose, sucrose, xylose, arabinose, ribose, mannose, galactose, fructose and mixtures thereof, advantageously glucose.

Furthermore, the invention also relates to the use of a metabolically engineered microorganism for glycine bioproduction or a salt or an ester thereof as described previously for the production and/or the secretion and/or the purification of acetyl-CoA.

The invention also relates to a method for producing glycine or one of its salts or esters comprising the following steps:
a) Cultivating a metabolically engineered microorganism according to the invention in a suitable culture medium comprising a carbon source to produce and accumulate glycine or any of the salts or esters in the culture medium and/or in the cells of the microorganism; and
b) Recovering the glycine, eventually present in the form of a salt, in particular ammonium, potassium or sodium, or one of its salts accumulated in the culture medium and/or in the cells of the microorganism.

In particular:
- the salt form of glycine is ammonium, potassium or sodium glycine salt; and
- the ester derived from glycine is selected from the group consisting of glycine ethyl ester, glycine methyl ester, glycine ethyl ester hydrochloride and glycine methyl ester hydrochloride.

Preferably, a subject of the present invention is a process for the production of glycine or one of its salts or esters as defined above having the following technical features, taken alone or in combination:
- the process further comprises a step c ) purification of glycine or one of its salts or esters;
- in step a) acetyl-CoA as a co-metabolite of glycine is produced and used as a substrate for the production of glycine, in particular by the glyoxylate shunt and the expression of an enzyme having glycine dehydrogenase activity as defined by EC 1.4.1.10, or of an enzyme having glyoxylate-alanine transaminase activity as defined by EC 2.6.1.4 or EC 2.1.6.40, to ensure the conversion of glyoxylate to glycine;
- the carbon source is a simple carbon source, advantageously a pentose or a hexose a disaccharide, more particularly chosen from the group consisting of glucose, sucrose, xylose, arabinose, ribose , mannose, galactose, fructose and mixtures thereof;
- the carbon source is glucose;
- the process of the invention is carried out in a bioreactor;
- the process of the invention is carried out in batch or fed-batch;
- The recovery of glycine or one of its salts or esters generally includes the steps of cell separation, as well as purification, concentration and drying of the product, respectively;
- ultrafiltration and centrifugation can be used to separate the cells from the fermentation medium;
- the addition of additives such as mineral acids or alkaline salts, or heating of the culture broth is implemented to optimize cell separation as described in documents US 5,017,480; WO 01/72689 or CN 108084041;
- various chromatographic methods of ion exchange can be applied for the separation of glycine or one of its salts before or after the elimination of the biomass, including in particular the use of primary cation exchange resins which facilitate the separation of the products according to their isoelectric point, where the resin is loaded with the solution, and the retained product is eluted separately after increasing the pH (for example by adding ammonium hydroxide) in the eluent; the use of ion exchange chromatography using fixed or simulated moving bed resins;
- different chromatographic steps may need to be combined in order to achieve adequate product purity;
- the purification process may also include a drying step which may involve any suitable drying means such as a spray granulator, a spray dryer, a drum dryer, a rotary dryer and a tunnel dryer;
- concentrated solutions of glycine or one of its salts or esters a mixture of them can be obtained by heating fermentation broths under reduced pressure with steam at 130°C using a general purpose concentrator or a thin film evaporator.

In the context of the invention, levels of production of glycine or of one of its salts or esters are expected at industrial levels, advantageously a concentration in the culture medium of the microorganism of the invention greater than or equal to 10 mg in 48 hours from 20g/L of carbon source, advantageously 20 g/L of glucose, in a fed-batch mode; preferably greater than or equal to 50mg, 100mg, 150mg, 200mg or 1 g in 48 hours from 20g/L of carbon source, advantageously of glucose, in a fed-batch mode; or even greater than or equal to 250 mg, 300 mg, 350 mg, 400 mg, 500 mg, 1 g, 1.25 g, 1.5 g, 2 g in 48 hours from 20 g/L of carbon source, advantageously 20g/L of glucose, in a fed-batch mode; or even greater than or equal to 5 g, 10 g, 15 g, 20 g, 25 g, 1 g, 50 g, 75 g, 100 g, or 150 g, 200g in 48 hours from 20 g/L of carbon source, advantageously 20 g/L of glucose, in a fed-batch mode. At these concentrations and under the conditions of the process according to the invention, glycine or one of its salts or esters remains soluble and does not exhibit any toxicity with respect to the microorganisms of the invention.

It appears from above that a microorganism according to the invention or implemented in a use or a method according to the invention is genetically modified to ensure efficient production and/or secretion at industrial levels of glycine or one of its salts or esters.

In addition, the production of glycine or one of its salts by fermentation brings the following technical and economic advantages:
- Process carried out at room temperature in aqueous solution using a renewable and inexpensive carbon source and at ambient pressure which is less energy consuming than high-pressure processes;
- No use of toxic products, organic solvents or petroleum-based resources; and
- Production of high purity glycine for the food, dietary supplement, chemical and pharmaceutical and cosmetic industries.

The present invention is illustrated without limitation by the following examples.

### EXAMPLES

### Example 1: Metabolic engineering of Escherichia coli for the production of glycine according to the invention

### 1. Materials & Methods

### a. Plasmid construction

All plasmids used in this study were constructed *via* the isothermal assembly reaction (Gibson *et al.,* 2009) using a commercial NEBuilder HIFI DNA assembly kit (New England Biolabs) according to the manufacturer's instructions. For construction of pMW12, the *kbl-tdh* operon of *E. coli* was amplified using primer pair MW15 & MW16 and assembled into plasmid pACT3 (Dykxhoorn *et al.,* 1996) at the *Sac*I and *Hind*III sites. For construction of pMW26/pMW27 the *E. coli* I-threonine aldolase gene *ItaE* wild type or that bearing mutation H126F was synthesized by IDT (Iowa, USA) and assembled into pACT3 between the *Sac*I and *Hind*III sites. For construction of plasmid pMW30/pMW31 bearing the synthetic operon *kbI-tdh-ItaE* or *kbI-tdh-ItaE-H126F,* the *kbl-tdh* and *ItaE* or *ItaE-H126F* were synthesized by IDT (Iowa, USA) as one operon bearing the RBS BBa_BB035 (Englund *et al.,* 2016) between *kbl-tdh* and *ItaE* or *ItaE-H126F* and assembled into pACT3 between the SacI and HindIII sites. *E. coli* TOP10 (Invitrogen) was used as host strain for plasmid construction. Plasmid pYN7 has been described elsewhere (see patent EP 0 152 830 A1 by Nakagawa et al, 1985). It has a pBR322 ori, ampicillin resistance gene Ap^{r}, and carries the *thrA^{∗}thrB thrC* gene under the control of its natural P_{thrLABC} promoter. The mutation in *thrA^{∗}* lead to the variant ThrA^{S345F} (Lee et al., 2007)

### b. Strain construction

Unless otherwise stated, the *E.coli* strain used in this work was BW25113. The *E. coli* mutant strains were constructed via P1 phage transduction. Preparation of P1 lysates and P1 phage transduction were performed as described elsewhere (Thomason *et al.*, 2007). Strains of the Keio collection (Baba *et al.*, 2006) bearing a single gene deletion and a FRT-site flanked kanamycin resistance cassette were used as donor strains. Successful transduction events were confirmed by colony PCR. FLP-recombinase flippase catalyzed excision of the antibiotic resistance marker was performed by transformation with the pCP20 plasmid (Cherepanov & Wackernagel, 1995) and subsequent verification of excision by colony PCR.

The *E. coli* strain 472T23 pYN7 (a K-12 background) was purchased at ATCCC under number 98081^{™}. It was constructed by (Kruse et al., 2002;Debabov, 2003D 6] and contains mutation at thrC^{∗} and ilvA^{∗} that lead to inactivation of the homoserine kinase and threonine deaminase and pYN7. Deletion of rthABC encoding threonine exporters {Livshits, 2003 #11032) and *gcvP* encoding glycine decarboxylase of the glycine cleavage pathway was carried out via PI transduction as described above.

### c. Glycine production in E. coli

For glycine production the plasmids pMW12, pMW26, pMW27, pMW30 and pMW31 were transformed into *E. coli* BW25113 or *E. coli* BW25113 ΔgcvP. Empty pACT3 was used as negative control. Pre-cultures of each transformed strain were grown o/n at 37°C and 200 rpm in 50 ml centrifuge tubes using 5 ml M9 minimal medium supplemented with 2 % (w/v) glucose and chloramphenicol (40 µg/ml). The next day, 25 ml M9 medium supplemented with 2 % (w/v) glucose and chloramphenicol (40 µg/ml) in 250 ml baffled Erlenmeyer flasks were inoculated with the pre-cultures to an initial OD₆₀₀ of 0.2 and were diluted to an initial OD600 of 0.05 - 0.2 and incubated at 37°C and 200 rpm. When an OD600 of 0.6 -1.0 was reached, cell cultures were induced by addition of IPTG to a final concentration of 1 mM. At several time points, 1 ml samples were taken. When required, 25 mM threonine was added to the M9 glucose medium at the same time as induction.

### d. Glycine detection by LC-MS

Glycine and threonine concentrations in the culture medium were determined on a Dionex ThermoFisher UltiMate 3000 system consisting of binary pumps, an on-line degassing unit, an autosampler, a column oven and a RS diode array detector (ThermoFisher Scientific, Waltham, USA) coupled to a ThermoFisher MSQ Plus mass spectrometer (ThermoFisher Scientific, Waltham, USA). Chromatographic separation was performed on a Poroshell 120 Hilic-Z column (2.1 mm × 150 mm, 2.7 µm P) maintained at 25 °C at a flow rate of 0.5 ml/min. The mobile phase consisted of Solution A (H₂O: 200 mM ammonium formate pH 3, 90%: 10 % (v/v)) and Solution B (acetonitrile: 200 mM ammonium formate pH 3, 90%: 10% (v/v)). A gradient elution program was optimized for the separation of glycine from threonine: 0 - 7 min, 95% - 80% Solution B, 7 - 7.5 min, 80% - 60% Solution B, 7. 5 - 10 min, 60% Solution B, 10 - 10.5 min, 60% - 95% Solution B, 10.5 - 14 min, 95% Solution B, with a total run time of 14 min. Samples were supplemented with L-Alanine-2,3,3,3-d4 as internal standard to a final concentration of 3.8 mM and subsequently diluted 1:20 in solution B. The sample injection volume were 10 µl. Data acquisition and analysis were performed with Dionex Chromeleon 7 (ThermoFisher Scientific, Waltham, USA).

### 2. Results

The results of the biotransformation of glucose into glycine by different strains cultured in a M9 medium containing 20 g/l of glucose for 48 hours are shown in Table 1.

| | | **Glycine [mg/l]** | |
|---|---|---|---|
| **Strain number** | **Genotype** | **Mean ± SD (w/O addition of threonine)^{∗}** | **Mean ± SD (with addition of 25 mM threonine)^{∗}** |
| Gly1 | BW25113 + pACT3 | bd | bd |
| Gly2 | BW25113 + pACT3_kbl-tdh (pMW12) | bd | 342 |
| Gly3 | BW25113 + pACT3_ItaE-H126F (pMW26) | bd | bd |
| Gly4 | BW25113 + pACT3_ItaE-WT (pMW35) | bd | bd |
| Gly5 | BW25113 + pACT3_kbl-tdh-ItaE-H126F (pMW31) | bd | 148 |
| Gly6 | Δgcvp + pACT3 | 28 | 332 |
| Gly7 | Δgcvp + pACT3_kbl-tdh (pMW12) | 355 | 1379 |
| Gly8 | Δgcvp + pACT3_ItaE-H126F (pMW26) | 406 | 1470 |
| Gly9 | Δgcvp + pACT3_ItaE-WT (pMW35) | 286 | 1029 |
| Gly10 | Δgcvp + pACT3_kbI-tdh-ItaE-H126F (pMW31) | 231 | 1020 |
| Gly11 | *suc*⁺, *relA+, ΔthrC, ilvA-, ΔrhtA, ΔrhtBC* pYN7 | bd | nt |
| Gly12 | *suc*⁺, *relA+, ΔthrC, ilvA-, ΔrhtA, ΔrhtBC, ΔgcvP* pYN7 | 278 | nt |
| Gly13 | *suc*⁺*, relA+, thrC-, ilvA-, ΔrhtA, ΔrhtBC, ΔgcvP,* pYN7 + pACT3 | 156 | nt |
| Gly14 | *suc*⁺*, relA+, thrC-, ilvA-, ΔrhtA, ΔrhtBC, ΔgcvP,* pYN7 + pACT3*-kbl-tdh* (pMW12) | 458 | nt |
| Gly15 | *suc*⁺*, relA+, thrC-, ilvA-, ΔrhtA, ΔrhtBC, ΔgcvP,* pYN7+pACT3-*Itae*-H126F (pMW26) | 1072 | nt |
| Gly16 | *suc*⁺*, relA+, thrC-, ilvA-, ΔrhtA, ΔrhtBC, ΔgcvP,* pYN7+ pACT3_kbI-tdh-ItaE-H126F (pMW31) | 311 | nt |

| | | | |
|---|---|---|---|
| Data shown are the mean of two biological replicates ^{∗}values are the mean + SD of two independent biological experiments; ^{S}bd = below detection which is < 0.2 mM glycine; ^{£}nt: not tested | | | |

Regarding metabolic engineering microorganism Gly2 to Gly5, expression of both threonine degradation pathway I and II does not promote glycine production. On the other hand, the removal of the *gcvP* gene which renders the glycine cleavage system (GCS) inactive that catalyzes the degradation of glycine into methyl-folate, CO₂ and NH₃ resulted in glycine producer Gly6 strain that can produce a low amount of glycine (28 mg glycine produced in 48 h from 20 g/l glucose).

As regards to the metabolically engineered microorganism number Gly7 that is deleted for the GCS in which the *tdh-kbl* pathway was overexpressed, the production of glycine was found to be significantly increased to levels in the range of 300 -500 mg/l glycine.

Likewise in strain Gly9 which also carries a deletion of GCS, but in which the threonine degradation pathway dependent on *ItaE* was overexpressed, the glycine titer at 48 h of growth on M9 containing 20 g/l glucose raised to 286 mg/l and this titer was even higher by expression of the *ItaE*^{∗} encoding the LtaEH126F variant. On the other hand, it appears that the overexpression of both *tdh-kbl* and *ItaE*H126F did not enhance glycine production as compared to the overexpression of *tdh-kbl* or *ItaE*H126F alone.

To evaluate that the promotion of threonine synthesis is critical in glycine production, two complementary experiments were carried out. On the one hand, exogenous threonine at 25 mM was added to culture of strain GLY1 to GLY10. Results show clearly an increased production of glycine as example in strain GLY8, the production of glycine is increase by 4 times in the presence of threonine as compared in the absence, supporting the fact that boosting threonine pathway is critically important in glycine production.

On the other hand, a threonine producing strain (472T23 pYN7) was used as a recipient for glycine production. In this recipient strain, the threonine operon is expressed from plasmid pYN7. pYN7 is a pBR322-based plasmid that carries *thrA^{∗}, thrB* and *thrC* under control of its natural promoter. In addition, *thrA^{∗}* gene contains a mutation that encodes the ThrAS345F variant which is no longer sensitive to feedback inhibition by threonine (Lee et al., 2007). In this strain, the threonine exporter genes *rhtA, rhtB* and *rhtC* were deleted (Gly11), to prevent export of threonine, as well as *gcvP* to inactivate the glycine cleavage system (Gly12). While Gly11 did not produce glycine, GLY13 produced 278 mg/l from 20 g/l of glucose within 48 h, supporting the importance of deleting the GCS in the glycine production.

Overexpression of *tdh-kbl* in Gly12 giving rise to Gly13 further increased glycine production to 454 mg/L glycine. Likewise for the overexpression of *ItaE^{∗}* encoding LtaEH126F variant in Gly13 giving rise to Gly14, which produced more than 1000 mg/L glycine in 48 h from 20 g/l glucose. On the other hand, it appears that production of glycine in Gly16 that overexpressed both pathways (*tdh-kbI* and *ltaE*) was not better or even lower than in strain Gly14 and Gly15 which overexpressed one of the two pathways individually.

### References

Baba, T., Ara, T., Hasegawa, M., Takai, Y., Okumura, Y., Baba, M., Datsenko, KA, Tomita, M., Wanner, BL, & Mori, H. (2006 ). Construction of Escherichia coli K-12 in-frame, single-gene knockout mutants: The Keio collection. In Molecular Systems Biology. (Vol. 2).
Boyd, WJ, & Robson, W. (1935). The synthesis of amino-acids: Piperidine and diethylamine as catalysts in the condensation of aromatic aldehydes with hydantoins. Biochem J. 29, 542-545.
Chen Z., Rappert S., Sun J., & Zeng AP. (2011). Integrating molecular dynamics and co-evolutionary analysis for reliable target prediction and deregulation of the allosteric inhibition of aspartokinase for amino acid production. J Biotechnol. 154(4):248-54.
Cherepanov, PP, & Wackernagel, W. (1995). Gene disruption in Escherichia coli: TcR and KmR cassettes with the option of Flp-catalyzed excision of the antibiotic-resistance determinant. In Gene. 158(1), 9-14.
Debabov, V.G. (2003). The threonine story. Adv Biochem Eng Biotechnol. 79, 113-136.
Ding Y., Svingen GF, Pedersen ER, Gregory JF, Ueland PM, Tell GS, & Nygård OK. (2015). Plasma Glycine and Risk of Acute Myocardial Infarction in Patients With Suspected Stable Angina Pectoris. J Am Heart Assoc. 5(1).
Dong, X., Quinn, P.J., & Wang, X. (2012). Microbial metabolic engineering for L-threonine production. Subcell Biochem. 64, 283-302.
Dykxhoorn, DM, St. Pierre, R., & Linn, T. (1996). A set of compatible tac promoter expression vectors. In Gene. 177(1-2), 133-136.
Englund, E., Liang, F., & Lindberg, P. (2016). Evaluation of promoters and ribosome binding sites for biotechnological applications in the unicellular cyanobacterium Synechocystis sp. CCP 6803. Scientific Reports. 6(1), 36640.
Farfan, MJ, Aparicio, L., & Calderon, IL. (1999). Threonine overproduction in yeast strains carrying the HOM3-R2 mutant allele under the control of different inducible promoters. Appl Environ Microbiol. 65, 110-116.
Fesko, K. (2016). Threonine aldolases: perspectives in engineering and screening the enzymes with enhanced substrate and stereo specificities. Appl Microbiol Biotechnol. 100, 2579-2590.
Gibson, DG, Young, L., Chuang, RY, Venter, JC, Hutchison, CA, & Smith, HO (2009). Enzymatic assembly of DNA molecules up to several hundred kilobases. Nature Methods. 6(5), 343-345.
Huo X, & Viola RE. (1996). Functional group characterization of homoserine kinase from Escherichia coli. Arch Biochem Biophys. 330(2):373-9.
Kruse, D., Kramer, R., Eggeling, L., Rieping, M., Pfefferle, W., Tchieu, JH, Chung, YJ, Jr Saier, MH, & Burkovski, A. (2002). Influence of threonine exporters on threonine production in Escherichia coli. Appl Microbiol Biotechnol. 59, 205-210.
Lee, KH, Park, JH, Kim, TY, Kim, HU, & Lee, SY. (2007). Systems metabolic engineering of Escherichia coli for L-threonine production. Mol. Syst. Biol. 3, 149.
Omori K., Imai Y., Suzuki S., & Komatsubara S. (1993). Nucleotide sequence of the Serratia marcescens threonine operon and analysis of the threonine operon mutations which alter feedback inhibition of both aspartokinase I and homoserine dehydrogenase I. J Bacteriol. 175(3):785-94.
Orten, JM, & Hill, RM. (1931). A Simple Method for the Preparation of Glycine. Journal of the American Chemical Society. 53, 2797-2799.
Plamann, MD, Rapp, WD, & Stauffer GV. (1983). Escherichia coli K12 mutants defective in the glycine cleavage enzyme system. Mol Gen Genet. 192:15-20.
Strecker, A. (1850). The synthesis of lactic acid and a new glycine homologue. Ann. Chem. Pharm. 75, 27.
Thomason, LC, Costantino, N., & Court, DL. (2007). E. coli Genome Manipulation by P1 Transduction. In Current Protocols in Molecular Biology. 1.17.1-1.17.8.
Tonouchi, N., & Hisao I. (2016) Present global situation of amino acids in industry. Amino Acid Fermentation. 3-14.
Usha, V., Jayaraman, R., Toro, JC, Hoffner, SE, and Das, KS. (2002). Glycine and alanine dehydrogenase activities are catalyzed by the same protein in Mycobacterium smegmatis: upregulation of both activities under microaerophilic adaptation. Can J Microbiol. 48, 7-13.
Wendisch VF. (2020). Metabolic engineering advances and prospects for amino acid production. Metab Eng. 58:17-34.
Woodall, CA. (2003). Plasmid Vectors. Methods in Molecular Biology 235.
Zeng, YZ, Li, L., & Demopoulos, GP. (2016) Process for glycine production by antisolvent crystallization using its phase equilibria in the ethylene glycol-NH4Cl-water system. Industrial & Engineering Chemistry Research. 55(8): 2426-2437.

## Claims

1. A metabolically engineered microorganism for glycine bioproduction or a salt or ester thereof, the genome of said microorganism comprises:
a. attenuation of the expression of genes encoding enzymes having glycine cleavage system activity as defined by E.C. 1.4.1.27, in particular enzymes having glycine decarboxylase activity as defined by E.C. 1.4.4.2 and an aminomethyltransferase activity as defined by E.C. 2.1.2.10; and
b. overexpression of genes encoding enzymes having L-threonine 3-dehydrogenase activity as defined by E.C. 1.1.1.103 and glycine C-acetyltransferase activity as defined by E.C. 2.3.1.29; and/or
c. overexpression of a gene encoding an enzyme having L-threonine aldolase activity as defined by E.C. 4.1.2.48 or a variant of this enzyme, EC 4.1.2.42 or EC 4.1.2.49.

2. The metabolically engineered microorganism according to claim 1, **characterized in that** its genome further comprises the attenuation of the expression of a gene encoding an enzyme having dihydrolipoyl dehydrogenase activity as defined by E.C. 1.8.1.4.

3. The metabolically engineered microorganism according to claim 1 or 2, **characterized in that** its genome further comprises the overexpression of genes encoding an enzyme having an acetylating aldehyde dehydrogenase activity as defined by E.C. 1.2.1.10.

4. The metabolically engineered microorganism according to one of the preceding claims, **characterized in that** its genome further comprises the overexpression of a gene encoding an enzyme having threonine synthase activity as defined by E.C. 4.2.3.1.

5. The metabolically engineered microorganism according to one of the preceding claims, **characterized in that** its genome further comprises the overexpression of a gene encoding:
- an enzyme having a glycine dehydrogenase activity as defined by E.C. 1.4.1.10; and/or
- an enzyme having a D-amino acid oxidase activity as defined by E.C. 1. 4. 99.-.; and/or
- an enzyme having a glyoxylate-alanine transaminase activity as defined by E.C. 2.6.1.44; and/or
- an enzyme having glycine C-acetyltransferase activity as defined by EC 2.3.1.29; and/or
- an enzyme having L-amino acid dehydrogenase activity as defined by EC 1.4.1.9.

6. The metabolically engineered microorganism according to one of the preceding claims, **characterized in that** its genome further comprises the attenuation of the expression of a gene encoding:
- an enzyme having D-serine/D-alanine/glycine transporter activity as defined byTCDB 2.A.3.1.7; and/or
- an enzyme having threonine efflux permease activity as defined by TCDB 2.A.76.1.2.

7. The metabolically engineered microorganism according to one of the preceding claims, **characterized in that** the variant of the enzyme having an activity L-threonine aldolase as defined by E.C. 4.1.2.48 is the variant *H126F* of the constituent enzyme of *Escherichia coli* or an equivalent variant of another microorganism.

8. The metabolically engineered microorganism according to one of the preceding claims, **characterized in that** its genome further comprises the attenuation, or even the suppression, of the expression of the genes coding for enzymes having an extracellular threonine efflux activity (i.e. RhtC) as defined by TCBD 2.A.76.1.2, in particular threonine/homoserine export activity (RhtA) as defined by TCDB 2.A.7.3.6, homoserine/homoserine Iactone/β-hydroxynorvaline efflux permease activity (RhtB) as defined by TCDB 2.A.76.1.1.

9. The metabolically engineered microorganism according to one of the preceding claims, **characterized in that**:
- the enzyme having glycine decarboxylase activity as defined by EC 1.4.4.2 is encoded by sequence set in forth in SEQ ID NO: 1 of *E. coli,* or any sequence sharing an identity of at least 90% with said sequence or corresponds to SEQ ID NO: 2 or any sequence sharing an identity of at least 90% with said sequence or corresponds to an equivalent enzyme having the same enzymatic activity in another microorganism;
- the enzyme having aminomethyltransferase activity as defined by EC 2.1.2.10 is encoded by sequence set in forth in SEQ ID NO: 3 of *E. coli,* or any sequence sharing an identity of at least 90% with said sequence or corresponds to SEQ ID NO: 4 or any sequence sharing an identity of at least 90% with said sequence or corresponds to an equivalent enzyme having the same enzymatic activity in another microorganism;
- the enzyme having an L-threonine 3-dehydrogenase activity as defined by EC 1.1.1.103 is encoded by sequence set in forth in SEQ ID NO: 5 of *E. coli,* or any sequence sharing an identity of at least 90% with said sequence or corresponds to SEQ ID NO: 6 or any sequence sharing an identity of at least 90% with said sequence or corresponds to an equivalent enzyme having the same enzymatic activity in another microorganism;
- the enzyme having an L-threonine aldolase activity as defined by EC 4.1.2.48 is encoded by sequence set in forth in SEQ ID NO: 7 of *E. coli,* or any sequence sharing an identity of at least 90% with said sequence or corresponds to SEQ ID NO: 8 or any sequence sharing an identity of at least 90% with said sequence or corresponds to an equivalent enzyme having the same enzymatic activity in another microorganism;
- the enzyme having dihydrolipoyl dehydrogenase activity as defined by EC 1.8.1.4 is encoded by sequence set in forth in SEQ ID NO: 9 of *E. coli,* or any sequence sharing an identity of at least 90% with said sequence or corresponds to SEQ ID NO: 10 or any sequence sharing an identity of at least 90% with said sequence or corresponds to an equivalent enzyme having the same enzymatic activity in another microorganism;
- the enzyme having an acetylating aldehyde dehydrogenase activity as defined by EC 1.2.1.10 is encoded by sequence set in forth in SEQ ID NO: 11 of *E. coli,* or any sequence sharing an identity of at least 90% with said sequence or corresponds to SEQ ID NO: 12 or any sequence sharing an identity of at least 90% with said sequence or corresponds to an equivalent enzyme having the same enzymatic activity in another microorganism;
- the enzyme having threonine synthase activity as defined by EC 4.2.3.1 is encoded by sequence set in forth in SEQ ID NO: 13 of *E. coli,* or any sequence sharing an identity of at least 90% with said sequence or corresponds to SEQ ID NO: 14 or any sequence sharing an identity of at least 90% with said sequence or corresponds to an equivalent enzyme having the same enzymatic activity in another microorganism;
- the enzyme having glycine dehydrogenase activity as defined by EC 1.4.1.10 is encoded by sequence set in forth in SEQ ID NO: 15 of *Streptomyces phaechromogenes,* or any sequence sharing an identity of at least 90% with said sequence or corresponds to SEQ ID NO: 16 or any sequence sharing an identity of at least 90% with said sequence or corresponds to an equivalent enzyme having the same enzymatic activity in another microorganism;
- the enzyme having D-amino acid dehydrogenase activity as defined by EC 1.4.99.1. is encoded by sequence set in forth in SEQ ID NO: 17 of *E. coli,* or any sequence sharing an identity of at least 90% with said sequence or corresponds to SEQ ID NO: 18 or any sequence sharing an identity of at least 90% with said sequence or corresponds to an equivalent enzyme having the same enzymatic activity in another microorganism;
- the enzyme having glyoxylate-alanine transaminase activity as defined by EC 2.6.1.44 is encoded by sequence set in forth in SEQ ID NO: 19 of *E. coli,* or any sequence sharing an identity of at least 90% with said sequence or corresponds to SEQ ID NO: 20 or any sequence sharing an identity of at least 90% with said sequence or corresponds to an equivalent enzyme having the same enzymatic activity in another microorganism;
- the H126F variant of the enzyme having L-threonine aldolase activity as defined by EC 4.1.2.48 corresponds to the amino acid sequence as shown in SEQ ID NO: 21 of *E. coli* or any sequence sharing an identity of at least 90% with said sequence or corresponds to SEQ ID NO: 22 or any sequence sharing an identity of at least 90% with said sequence or corresponds to an equivalent enzyme having the same enzymatic activity in another microorganism;
- the enzyme having threonine/homoserine exporter activity as defined by TCDB 2.A.7.3.6 is encoded by sequence set in forth in SEQ ID NO: 27 of *E. coli,* or any sequence sharing an identity of at least 90% with said sequence or corresponds to SEQ ID NO: 28 or any sequence sharing an identity of at least 90% with said sequence or corresponds to an equivalent enzyme having the same enzymatic activity in another microorganism;
- the enzyme having homoserine/homoserine lactone/β-hydroxynorvaline efflux permease activity is encoded by sequence set in forth in SEQ ID NO: 29 of *E. coli,* or any sequence sharing an identity of at least 90% with said sequence or corresponds to SEQ ID NO: 30 or any sequence sharing an identity of at least 90% with said sequence or corresponds to an equivalent enzyme having the same enzymatic activity in another microorganism.

10. The metabolically engineered microorganism according to one of the preceding claims, **characterized in that** its genome further comprises:
- increasing the expression of at least one of the enzymatic activities selected from the group consisting of phosphoenolpyruvate carboxylase, isocitrate lyase, pyruvate carboxylase, and hexose symporter permease; and/or
- decreasing the expression of at least one of the enzymatic activities selected from the group consisting of lactate dehydrogenase, alcohol dehydrogenase, acetate kinase, phosphate acetyltransferase, pyruvate oxidase, isocitrate lyase, fumarase, 2-oxoglutarate dehydrogenase, pyruvate kinase, malic enzyme, phosphoglucose isomerase, phosphoenolpyruvate carboxylase, phosphoenolpyruvate carboxykinase, pyruvate-formate lyase, succinic semialdehyde dehydrogenase, sugar-transporting phosphotransferase, ketohydroxyglutarate aldolase, homoserine-O-succinyl transferase, homoserine kinase, homoserine efflux transporter, diaminopimelate decarboxylase, and/or methylglyoxal synthase.

11. The metabolically engineered microorganism according to one of the preceding claims, **characterized in that** it is :
- a bacteria, preferably of the family of *Enterobacteriaceae* or *Corynebacteriaceae,* preferably of the genus *Escherichia, Pantoea, Corynebacterium* or *Brevibacterium,* more particularly of the species *Escherichia coli, Pantoea ananatis* or *Cornebacterium glutamicum*; or
- a *fungi,* preferably of the family of *Ascomycota,* preferably of the group of *Saccharomyceta* or *Taphrinomycotina,* more particularly of the subphylum *Saccharomycotina, Pezizomycotina* or *Archaeorhizomycetes,* more preferably of the class *Saccharomycetes, Eurotiomycetes, leotiomyceta* , *Pezizomycetes* or *Archaeorhizomycetales,* preferably of the species *Saccharomyces cerevisiae, Talaromyces versatilis, Aspergillus niger.*

12. The metabolically engineered microorganism according to one of the preceding claims, **characterized in that** it is *Escherichia coli* in the genome of which:
- at least one of the genes selected from the group consisting of ppc, pck, aceA, galP, asd, thrA, metL, lysC all from *Escherichia coli*; pycA from *Lactococcus lactis,* pycE from *Corynebacterium Glutamicum* is overexpressed; and/or
- at least one of the genes selected from the group consisting of IdhA, adhE, ackA, pta, poxB, focA, pfIB, sad, gabABC, sfcA, maeB, ppc, pykA, pykF, mgsA, sucAB, ptsl, ptsG, pgi, fumABC,aIdA, IIdD, icIR, metA,, lysA, eda, rthA, rthB, rthC is deleted.

13. Use of a metabolically engineered microorganism according to one of claims 1 to 12 for the production of glycine or one of its salts or esters.

14. A process for producing glycine or any of its salts or esters comprising the following steps of:
a) Cultivate a metabolically engineered microorganism according to one of claims 1 to 12 in a culture medium comprising a carbon source to produce and accumulate glycine or one of salts or esters in the culture medium and/or in the cells of said microorganism; and
b) Recover the glycine or one of its salts accumulated in the culture medium and/or in the cells of said microorganism.

15. The process according to claim 14, **characterized in that** it further comprises a step c) of purification of glycine or one of its salts or esters.

16. The process according to claim 14 or 15, **characterized in that** the carbon source is a pentose or a hexose or a disaccharide, advantageously selected from the group consisting of glucose, sucrose, xylose, arabinose, ribose, mannose, galactose, fructose and mixtures thereof, preferably glucose.
